# EUROPEAN PATENT APPLICATION

(11) **EP 1 536 013 A1**
(43) Date of publication of application: **01.06.2005**
(21) Application number: 02783758.2
(22) Date of filing: 03.12.2002
(51) Int. Cl.: C12N 15/51, C12N 5/10, C12N 1/15, C12N 1/19, C12N 1/21, C07K 14/005, C07K 16/08, C12P 21/02, G01N 33/53

(54) **DNA SEQUENCE USEFUL IN DIAGNOSING HEPATITIS AND POLYPEPTIDE ENCODED THEREBY**

(30) Priority: 13.02.2002 JP 2002035161
(71) Applicant: Arima, Terukatsu, Kagoshima 892-0848 (JP)
(72) Inventor: ARIMA, Terukatsu, Kagoshima-shi, Kagoshima 892-0848 (JP); ISHIBASHI, Kazuaki, Kagoshima-shi, Kagoshima 890-0075 (JP); WANG, Hong, Kagoshima, Kagoshima 890-0065 (JP)
(74) Representative: Wablat, Wolfgang, Dr. Dr.
(86) International application number: PCT/JP2002/012679
(87) International publication number: WO 2003/068973

(57) **Abstract**

A novel cDNA clone-K, has been isolated and characterized as encoding a previously unknown polypeptide antigen recognized by antibodies in the serum of certain patients with chronic non-B non-C hepatitis or acute non-A non-B non-C non-D non-E non-G (NA-G) hepatitis. Hepatitis relating to anti-K-polypeptide antibodies in the patients' serum was disclosed to distribute worldwide and the antibody test for donated blood will be required to prevent blood transmission of the disease. The nucleic acid sequence is not represented in the genome of the hepatitis A virus (HAV), hepatitis B virus (HBV), hepatitis C virus (HCV), hepatitis D virus (HDV), hepatitis E virus (HEV) and hepatitis G virus (HGV). Neither is the nucleic acid sequence represented in the human genome. The data suggest that the RNA sequence corresponding to clone-K is contained within the genome of an external infective agent other than HAV, HBV, HCV, HDV, HEV and HGV, and therefore may represent an additional etiologic agent or contributing factor to the development of NA-G hepatitis in human patients. The K-nucleotide and corresponding polypeptide and respective variants thereof will be useful in a variety of procedures directed at prevention, diagnosis and treatment of NA-G hepatitis.

SEQUENCE LISTING

<110>  Arima, Terukatsu
<120>  A DNA sequence and encoded polypeptide useful in the diagnosis of
        hepatitis disease
<130>  SAP-690-PCT
<140>  JP 200235161
<141>  2002-02-13
<150>  JP 200235161
<151>  2002-02-13
<160>  2
<170>  PatentIn version 3.2

<210>  1
<211>  69
<212>  PRT
<400>  1

<210>  2
<211>  207
<212>  DNA
<400>  2
ttcgcggccg cgtcgacggg aagcaggccc gcgctctcga cctcggaggc ctcgggccgc      60

cccagcccgg cgcgccccga tcagctactc accgttggcg cgtttgaggg cattttccgg     120

cctctgaaaa taggccggca tcttggcggc aggctcagct cacccggcgt cagcgaactc     180

tctagtggcc gtcgacgcgg ccgcgaa                                         207

## Description

### FIELD OF THE INVENTION

There are six human hepatitis viruses, hepatitis A virus (HAV), hepatitis B virus (HBV), hepatitis C virus (HCV), hepatitis D virus (HDV), hepatitis E virus (HEV) and hepatitis G virus (HGV), although HGV does not appear to cause hepatitis. This invention relates to a DNA sequence and encoded polypeptide, wherein the encoded polypeptide represents an antigen specifically recognized by antibodies in certain patients with chronic non-C, non-B (NBNC) hepatitis. Consequently, the peptide is also specific to acute non-A, non-B, non-C, non-D, non-E and non-G (NA-G) hepatitis as among these acute hepatitis only B and C progress to chronic hepatitis. The nucleic acid and encoded polypeptide of the present invention will be useful in a variety of assays for NA-G hepatitis.

### BACKGROUND OF THE INVENTION

Viral hepatitis is a major public health problem, and the most common cause of liver diseaseworldwide. Annually, 300, 000 cases of acute viral hepatitis occur in the United States, fortunately only rarely resulting in fulminant hepatitis, but both hepatitis B and C give rise to thousands of cases of chronic viral hepatitis. There are 5 million people afflicted with chronic viral hepatitis, unquestionably the most common cause of chronic liver disease. Although the incidence of acute viral hepatitis is decreasing throughout the world with more effective public health measures and implementation of hepatitis B vaccine programs, each new case of acute viral hepatitis C has an 85% chance of being added to the growing pool of chronic viral hepatitis. Over 15,000 Americans (up to 40, 000 Japanese) die each year as a consequence of chronic viral hepatitis, and as cases of chronic viral hepatitis C age, it is estimated that the annual mortality rate in the next two decades will approach 25,000. The annual cost of viral hepatitis is enormous, in the billions of dollars, and this does not take into account the intangibles of impaired quality of life.

As cited supra there are six known human hepatitis viruses, hepatitis A, B, C, D, E, and G viruses, with diverse genetic composition and structures. They have ribonucleic acid (RNA) genomes except for HBV that has deoxynucleic acid (DNA) genome. HBV utilizes a RNA intermediate to replicate a deoxyribonucleic acid (DNA) genome. HDV requires the HBV surface antigen for its viral assembly and infectivity as described by Doo et al. , Schiff et al. eds., supra, pages 725-744 (1998). HGV does not appear to cause hepatitis. Tan et al., J Infect Dis 179: 1055 (1999).

### Parenteral Transmission of Hepatitis Viruses

Parenteral transmission routes for hepatitis viruses is well known as reviewed by Sjogren, Chan et al., Davis, Rizzetto et al., Krawczynski et al. and Schiff, Schiff et al. eds., supra, pages 745-869 (1998). Known hepatitis viruses, HAV, HBV, HCV, HDV, HEV and HGV can parenterally be transmitted. Their transmission routes are:
Hepatitis A: The primary routes; fecal-oral, person-to-person contact, or ingestion of contaminated food or water. Sexual transmission is prominent among homosexual men. Although rare, transmission by parenteral route has been documented following blood transfusion or use of blood products.
Hepatitis B: Transfusion, sexual, percutaneous and perinatal, health care environment and transplantation. Others include close bodily contact, blood-feeding insects like mosquitoes and various body secretions such as semen and saliva.
Hepatitis C: Transfusions, transplantation or sharing illicit hepatitis C: Large or repeated percutaneous exposures such as through transfusions, transplantation from infected donor or sharing illicit drug paraphernalia, exposure to infected contacts through sexual activity, household contacts, perinatal exposure, and perinatal exposures in the health care setting.
Hepatitis D: Through the same modalities of HBV transmission, i.e., by the parenteral route either overt or covert.
Hepatitis E: The fecal-oral route is the predominant mode of transmission. HEV is a non-enveloped RNA virus that is responsible for large epidemics of acute hepatitis and a proportion of sporadic hepatitis cases in southeast and central Asia, the Middle East, parts of Africa, and Mexico. Some reports have suggested parenteral transmission of HEV from its association to HBV or HCV infection, or due to the development of hepatitis E after blood transfusion. Hepatitis G: Although the role of HGV in causing hepatitis is poorly defined and HGV does not appear to cause hepatitis as described supra, HGV is transmitted parenterally, sexually and perinatally.
Sixth (Non-A, Non-B, Non-C, Non-D, Non-E and Non-G: NA-G) Hepatitis Viruses
In the past thirty years, five hepatitis agents (HAV, HBV, HCV, HDV and HEV) have been discovered, and there is evidence for at least one additional virus. Purcell, Proc Natl Acad Sci USA 91: 2401 (1994) . As mentioned supra so-called HGV does not appear to cause hepatitis, NA-G virus occupies the sixth position of the hepatitis viruses.
Sporadic Acute NA-G Hepatitis Suggesting Blood Transmission Stored sera of 57 patients hospitalized between 1971 and 1974 with acute hepatitis, designated at that time as non-A non-B (NANB) hepatitis, were reported to be re-studied for the presence of anti-HAV IgM, hepatitis B surface antigen (HBsAg), anti-HCV IgG, and anti-HEV IgG. Sera from 27 (47%) of the NANB hepatitis patients were negative on all tests. These NA-G hepatitis cases included children and elderly adults, but as a group were significantly more likely to have used intravenous drugs than hospitalized control patients. Binotto et al., Aust N Z J Med 30: 668 (2000).
Transfusion Associated Chronic NBNC Hepatitis
   We are now at a time when the incidence of acute transfusion associated hepatitis (acute TAH) is approaching zero and is almost certainly under 0. 5% per transfusion episode as reported by Alter, and that TAH has been virtually eliminated, and any improvement in safety will be very small and will require huge costs as described by Gerlich et al..
However, there are evidence suggesting the presence of chronic NA-G TAH shown infra. Persons with NANB hepatitis (cases) identified in 5 transfusion studies in the early 1970s have been followed ever since and 39 of 129 (30%) living persons with previously diagnosed TAH were unrelated to hepatitis viruses A, B, C, and G, suggesting the presence of another unidentified agent in these patients in their chronic state, chronic NBNC hepatitis.
New Candidates (TTV and SENV) for Hepatitis Virus but not Causing Hepatitis
   A possible hepatitis virus, TT virus (TTV), was discovered in the blood of a patient with post-transfusion NA-G hepatitis. However TTV has no association to hepatitis and may not be a primary hepatitis virus. Furthermore TTV infection was common in patients on hemodialysis but was not associated with liver disease.
Most recently, a DNA virus designated as SEN virus (SENV), has been announced as a maj or cause of NA-G hepatitis. This new virus was present in nearly 2% of US donors and the vast majority of SENV-infected recipient did not develop hepatitis. Another study on the existence of SENV in 378 Japanese patients with liver diseases did not suggest SENV as a possible causative agent of hepatitis.

### Chronic Viral Hepatitis is at the Highest Risk Factor of Primary Liver Cancer

Chronic hepatitis is largely responsible for primary liver cancer as reviewed infra.

Estimated number of cases: In 1985, 315, 000 new cases of primary liver cancer became apparent worldwide, accounting for 4.1% of all human cancer cases. The largest concentration of cases is found in Asia; these represent 70% of all primary liver cancer cases in the world. Africa is second in terms of number of cases, accounting for 37,600 cases. Japan accounts for close to 23000 cases per year. In North America close to 7,000 cases per year occur; there are 30, 000 cases in Europe and 12, 000 cases in the countries of the former Soviet Union. Australia, New Zealand, and the Pacific Islands account for fewer than 1,000 cases per year as reviewed by Bosch supra, pages 13-14 (1997).

Rank of primary liver cancer: In terms of relative frequencies over the estimated total number of cancer cases, if each primary site is taken separately, primary liver cancer ranks fifth. In the developing countries, primary liver cancer is, by all estimates, the third most common cancer among men after stomach and lung cancers as reviewed by Bosch supra, page 14 (1997).

Mortality of primary liver cancer : The mortality/incidence ratio reported by the cancer registries is close to or greater than 1, indicating that the majority of cases do not survive 1 year. This is confirmed by most clinical series, particularly in developing countries. In Europe, relatively high mortality rates (>5.0 per 100,000) are seen in Italy, Spain, France, Switzerland, and some Eastern European countries. The highest mortality rates are seen in Greece (16.1 and 7.8 per 100, 000 in men and women, respectively), a country without established cancer registries as reviewed by Bosch supra, pages 15-16 (1997).

Primary liver cancer incidence: The areas at higher risk are the coastal areas of Southeast Asia, China, Japan, the sub-Saharan African countries, and Melanesia. Areas at intermediate risk are the northern Mediterranean countries and the lowest risk is recorded in countries in northern Europe, most of the Americas and Australia as reviewed by Bosch supra, page 16 (1997).

Trends in primary liver cancer: In 96 cancer registries, two recent time intervals (1978 to 1982 and 1983 to 1986) have been chosen to evaluate trends while limiting the variability induced by registration and diagnostic practices. In Europe, an increase in Scandinavia has been reported. In Japan, there is a 15% to 20% increase in the recorded incidence of primary liver cancer. The increasing trend in Japan has been documented since the early 1970s and has been largely attributed to massive exposure of the population to HCV through transfusion or contaminatedneedles in the early 1960s . Other registries suggesting increasing trends include those in Australia, Canada (males), and several ethnic groups in the United States. Moderate increases, particularly among men, have been recorded in France, Italy, and some registries in the United Kingdom. A decrease in the primary liver cancer AAIRs (age-adjusted incidence rates) has been reported in several registries in India, Israel, Spain, and Latin America. Exposure to HBV in early life in high-risk countries (largely from infected mothers) may account for the early onset and rise in incidence, as well as the early occurrence of the male excess. Other risk factors, such as HCV, alcohol, oral contraceptives, and tobacco, may account in part for the late onset cancers, the progressive increase in incidence with age, and the later peak in the male excess as reviewed by Bosch supra, pages 20-21 (1997).

Risk factors and consistency with the uneven distribution of primary liver cancer: In developed populations at low risk for primary liver cancer in the United States, Europe, and Japan, HBV and HCV may account for 70% to 75% of primary liver cancer cases. Therefore NA-G hepatitis may contribute to the rest, up to 20% of primary liver cancer cases in these regions. In high-risk countries in Southeast Asia, China and sub-Saharan Africa, HBV is highly prevalent and responsible for more than 60% of the primary liver cancer cases. HCV may play a minor role in these areas, perhaps in fewer than 10% of cases. Oral contraceptives may act as a co-factor or a promoter of the carcinogenic process of HCV and HBV. Lack of reliable techniques to assess individual exposure over long periods still limits our ability to quantify the risk of aflatoxin as reviewed by Bosch supra, pages 23-25 (1997).

### Incidence of NA-G Virus Related Liver Diseases in Applicant's Clinic

In 1998 the applicants saw 633 patients with chronic liver diseases as shown in Table 1. They were tested for HBV-DNA , HCV-RNA, HTLV-1-RNA and HIV-RNA. Liver histology of patients with chronic NBNC hepatitis was compatible with features of chronic viral hepatitis. Approximately 10-20% of the patients with chronic liver diseases are NBNC origin, that consequently are NA-E or NA-G origin as cited infra (Table 1).

**Table 1**

| Patients with Histology Proven Chronic Liver Diseases Seen by Applicants' in 1998 | | | | |
|---|---|---|---|---|
| | | | Cause | |
| Diseases | Number of Patients | HBV | HCV | Unknown* |
| Chronic Hepatitis | 546 | 65(11.9%) | 414(75.8%) | 67(12.3%) |
| Liver Cirrhosis | 28 | 4(14.3%) | 16(57.1%) | 8(28.6%) |
| Primary Liver Cancer | 59 | 7(11.9%) | 42(71.2%) | 10(16.9%) |
| Total | 633 | 76(12.0%) | 472(74.6%) | 85(13.4%) |

| | | | | |
|---|---|---|---|---|
| ^{*}Negative for HBV-DNA and HCV-RNA (NBNC=NA-G), and liver histology is compatible with chronic viral hepatitis. | | | | |

Non-neutralizing Antibodies Targeting Hepatitis Virus Antigens Serum non-neutralizing antibodies against 2 antigens coded by HBV genome and expressed in the infected liver cells, an intracellular antigen (HBc-antigen: HBcAg) and another secretory antigen (HBe-antigen: HBeAg) associated with HBV replication, are well-known to be detected in patients with on going HBV hepatitis or in patients ceasing hepatitis. Similarly, antibodies against non-neutralizing antigens, including a variety of structural and non-structural proteins coded by HCV genome and expressed in the infected liver cells, can be detected in the patients throughout the course of HCV infection. Chan et al. and Davis, Schiff et al. , eds., supra, pages 757-791 and pages 793-836 (1998). Therefore similar phenomena in patients with NA-G hepatitis can easily be predicted.

### Circulating Hepatic mRNA in Patients with Liver Diseases and Healthy Controls.

In a study, total RNA was extracted from the lymphocytic layer of peripheral venous blood of caesium chloride gradient centrifugation. The mRNA was reverse transcribed, and hepatic lipase cDNA was amplified by hepatic lipase specific primers with RT-PCR.

Amplificates were visualized in samples from patients with primary liver cancer and normal controls by agarose gel electrophoresis and ethidiumbromide staining. Leonhardt et al., Eur J Surg 165: 539 (1999). Similarly, in the majority of patients with acute and chronic liver disease, albumin-mRNA-positive cells in their peripheral blood mononuclear cell fraction were detected by RT-PCR. Muller et al., Hepatology 25: 896 (1997). Furthermore, alpha fetoprotein mRNAs, which is mainly seen in liver cells, were frequently detected by the same reaction, in peripheral blood cells from patients with liver diseases and healthy volunteers. Ishikawa et al., Jpn J Clin Oncol 28: 723 (1998). The evidence suggest that mRNAs deriving from replicating NA-G hepatitis virus in some tissues including liver, can be predicted to be in the peripheral blood in patients with NA-G hepatitis.

### Sensitivity of Antibody Tests Diagnosing Hepatitis

Screening tests for HAV and HBV made it possible in the mid-1970s to examine cases of transfusion-associated hepatitis (TAH) and to demonstrate that only approximately 25% resulted from HBV and that none were related to HAV. Consequently, approximately 75% of TAH became classified as non-A, non-B (NANB) hepatitis. The NANB hepatitis agent remained a virologic enigma until researchers at the Chiron Corporation developed a single antigen first-generation enzyme immunoassay (EIA-1) in 1989 for the detection of hepatitis C virus (HCV) infection. Retrospective analysis of pedigreed samples at the National Institute of Health (NIH) showed that 70% to 90% of NANB hepatitis cases were HCV related by using EIA-1 antibody targeting HCV antigens for the screening of donors blood. The impact of HCV blood donor screening has been enormous. The EIA-1 prevented 40,000 HCV infections within the first year, and the second-generation assay (EIA-2) has actually reduced new transfusion-related HCV infections to almost zero. Alter, Am J Med 107: 16S (1999).

### Applicant's Experience of Molecular Cloning of NANB- and NANBNC-Viruses

Molecular cloning of RNA found in the plasmas of a chimpanzee infected with NANB post-transfusion hepatitis had shown the presence of a viral genome of about 10kb Choo et al., Science 244: 359 (1989), . Similarly, one of applicants of this invention, Arima, had isolated clones relating to NANB hepatitis, from patients with NANB hepatitis. Arima and Fukai, EPO Application No. 89309261.9 (1989). Most of NANB post-transfusion cases had been shown to be associated with infection by hepatitis C virus (HCV) which has been widely accepted as a strong diagnostic marker for NANB post-transfusion hepatitis. However it is still unclear whether other viral agents can cause this syndrome. Several researchers, including one of the present inventors (Terukatsu Arima), have identified cDNA sequences encoding polypeptides reactive with sera from NANB hepatitis patients that are not represented in the known sequences of HCV and its variants. Arima et a1., Gastroenterology Jpn 24: 540 (1989); Arima et al., Gastroenterology Jpn 24: 545 (1989); Arima et a1., Gastroenterology Jpn 24: 685 (1989); Arima et a1., Gastroenterology Jpn 25: 218 (1990). These cDNAs apparently are not encoded by the human genome, and therefore are not host-specified responses to the hepatitis disease state.

### SUMMARY OF THE INVENTION

Most recently, among human hepatitis viruses, only HBV and HCV are known to cause chronic hepatitis. However, another unidentified agent inducing chronic hepatitis has been suggested by the applicants and a review published in USA as cited infra.

Structural and soluble components of viruses responsible for chronic hepatitis and antibodies against the component proteins of the new viruses co-exist in the serum of the patients with the disease as stated supra. In addition, mRNAs, at least their fragments, of albumin, alpha fetoprotein (AFP) and liver specific lipase transcribed in the liver cells and other cells are known to be released to the peripheral blood of the patients as well as normal subjects as cited supra. Consequently it is easily predicted that mRNAs transcribed from the viral genome at their replication in the liver cells are released in the blood.

Therefore the applicants prepared RNA, by physical and chemical procedures, from mononuclear cells, plasma and serum fractions of patients with chronic NBNC hepatitis, negative for both of HBV-DNA and HCV-RNA. In these fractions NA-G hepatitis virus and virus-like particles, and virus derived mRNAs from the infected cells including liver cells are predicted to be present in peripheral blood of the patients. Extracted nucleic acids were converted into double-stranded complementary DNA (cDNA) and introduced into an expression vector, a derivative of bacteriophage lambda, λgt11. The λgt11 containing recombinant DNA from chronic NBNC hepatitis patients were then screened for the production of recombinant-encoded proteins reactive with antibodies contained within the blood of chronic NBNC hepatitis patients by plaque immunoscreening as described infra.

One recombinant phage, designated K, was found to contain a recombinant DNA insert that encodes a polypeptide sequence specifically reacting with more than 70% of sera obtained from chronic NBNC hepatitis patients.

Western blot to probe interaction between glutathione-S-transferase (GST) fused clone-K protein expressed by E. coli and a serum positive for clone-Kprotein specific (anti-K) antibodies by plaque immunoscreening identified a single reactive band at an predicted molecular size of GST fused clone-K protein.

By plaque immunoscreening, this polypeptide antigen expressed by the recombinant λt11 phage in bacteriolysis plaques on a sheet of E. coli growing on a agar gel and blotted on a filter sheet, can detect antibodies present in the blood of 72% of chronic NBNC hepatitis patients, 27.6% of acute NA-G hepatitis patients and 14.5% of maintenance hemodialysis patients, 12.5% of blood donors with elevated ALT level and up to 23% of persons having history of blood transfusion. Although anti-K antibody hereinafter "anti-K", was rarely detected in asymptomatic random blood donors its utility for testing donated blood to prevent transfusion associated hepatitis was suggested supra and infra..

Worldwide distribution of anti-K was assessed in Japan, Taiwan, Korea, USA, Brazil, Germany, Italy, Greece and Czech Republic. Therefore this antigen, hereinafter "K-polypeptide", appears to have utility in the detection and screening of the disease at least by plaque immunoscreening.

The nucleotide sequence of clone-K and its encoded polypeptide are related to the virus thought to cause chronic NBNC hepatitis. In addition, the nucleotide sequence of clone-K does not appear to be contained within human chromosomal DNA. This later result suggests that clone-K is contained within the genome of an external infective agent associated with NA-G hepatitis other than known hepatitis viruses, and therefore may represent an additional etiologic agent or contributing factor to the development of chronic NBNC hepatitis consequently as well as acute NA-G hepatitis in human patients.

In other aspects of the present invention, the applicants describe a variety of immunodiagnostic and probe assays for the detection of anti-K-polypeptide antibodies and K-related nucleic acids in a patient sample. The K-polypeptide in question is produced through expression in compatible host cells of recombinant bacteriophage or recombinant plasmid vectors, or through chemical synthesis.

Vaccines for protection against the agent represented by clone-K are formulated as compositions including one or more of the immunodiagnostic epitopes represented in the K-polypeptide. Moreover, polyclonal and monoclonal antibodies directed against one or more of the immunodiagnostic epitopes of the K-polypeptide are readily produced using techniques known to those skilled in the art. Such antibodies, or active fragments thereof, are useful as passive immunization against the agent represented by clone-K.

Additionally, DNA sequences flanking those described within this invention will have utility in the diagnosis of NA-G hepatitis and other diseases. These additional sequences can be easily isolated using standard techniques given the sequence of clone-K.

### BRIEF DESCRIPTION OF THE DRAWINGS

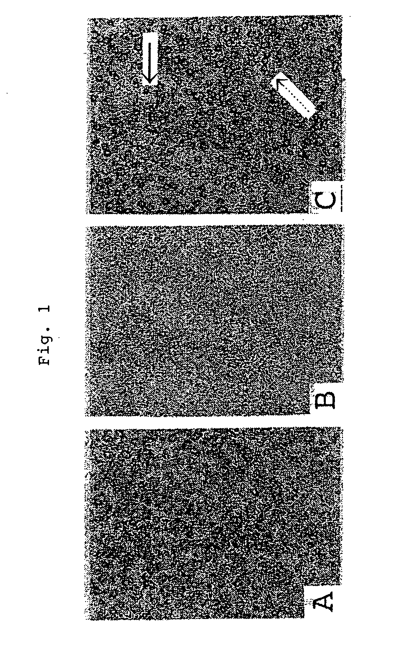

Figure 1: Plaque Immunoscreening
   (A) A membrane lifted from a bacteriological plate containing E. coli infected with λgt11 clone-K, andreactedwithserumpooled from patients suffering from chronic NBNC hepatitis. Antibody binding to the membrane is detected by a chromogenic (4-chloro-1-naphtol) enzyme reaction, resulting in a dark blue spot.
   (B) A membrane lifted from a bacteriological plate containing E. coli infected with wild-type λgt11 (no recombinant) and reacted with the same serum as described in Figure 1(A). Antibody binding to the membrane can' t be detected by a chromogenic enzyme reaction seen in Figure 1 (A).
   (C) A membrane lifted from a bacteriological plate containing an equal mixture of E. coli infected with λgt11 clone-K used in (A) and wild-type λgt11 used in (B), and reacted with the same serum as described in (A) and (B), resulting a mixture of dark blue spot and light blue spot. The membrane can be used for the detection of anti-K antibodies in test samples (Plaque Immunoscreening). In this immunodiagnostic format, a positive test is indicative of the presence of a patient's anti-K-polypeptide antibody sandwiched between the immobilized K-polypeptide and the signal antibody. In the absence of anti-K-polypeptide antibody (negative test), the antigen-antibody-signal antibody complex does not form and no chromogenic reaction above background is detected.

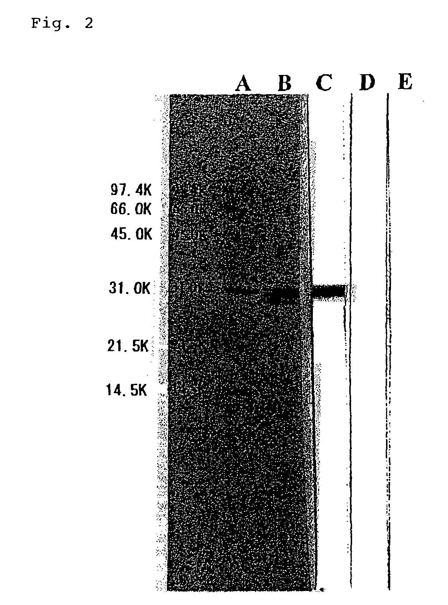

Figure 2: Identification of Anti-K by Western Blot Procedure
   (A) Low molecular weight markers (Amersham Corp., Arlington Heights, IL) separated by SDS-polyacrylamide gel electrophoresis (PAGE) and stained by CBB.
   (B) Purified and solubilized glutathione-S-transferase (GST) clone-K fusion protein run in an adjacent lane to well (lane A) was found to be identical to the predicted molecular weight (32KD) of the fusion protein.

   The other 3 lanes of the fusion protein run on the same plate were transferred to a polyvinylidene difluoride (PVDF) filter and cut along the lanes. The cut filters (lanes C, D and E) were subsequently exposed to serum antibodies separately. The bound antibody was stained by the second antibody, anti-human IgG+IgM antibodies, coupled to horseradish peroxidase and followed by enzymatic production of blue color.
(C) Exposed to serum antibody from a patient positive for anti-K by plaque immunoscreening showing a single brown-colored (Diaminobenzidine: DAB) bandatapredictedmolecularsize (32KD) of the GST-clone-K fusion protein.
(D and E) Exposed to 2 control sera negative for anti-K by plaque immunoscreening run adjacent to lane (C) where no brown-colored band is detected.

### DETAILED DESCRIPTION OF THE PREFFERED EMBODIMENT

A polypeptide having diagnostic utility for chronic NBNC hepatitis, consequently NA-G hepatitis, and representing a nucleic acid and amino acid sequence having no detectable relationship to any other described NA-G hepatitis agent would represent a significant advance over the prior art. Such a polypeptide, as well as the corresponding nucleic acid, would be of great value in the ongoing effort to develop a complete preventative and diagnostic regimen for NA-G hepatitis. The present invention encompasses such a polypeptide and its corresponding nucleic acid sequence.

### Isolation of Clone-K Specific for Chronic NANB Hepatitis, Consequently for NA-G Hepatitis

RNA was isolated from pooled peripheral mononuclear blood cells (PMBC), plasma and serum obtained from 5 patients displaying the clinical and histological picture of chronic NBNC hepatitis. These pooled PMBC, pooled plasma and pooled serum were separated from whole blood according to Ting et al., VoxSang20: 5 61 (1971). They were supposed to be enriched for virus and virus-like particles. RNAwas extracted from the musing a commercial product, Trizol Reagent (Life Technologies, Gaithersburg MD, USA) as reviewed by Sambrook et a1.. Construction of cDNA library from the RNA was achieved by using Superscript and Superscript III from Life Technologies according to Sambrook et al.. The cDNA was cloned into the EcoRI site of the phage expression vector λgt11 using Gigapack III Gold (Stratagene, La Jolla, CA, USA) according to Sambrook et al..

Chromogenic screening (plaque immunoscreening) of expression libraries constructed in bacteriophage λgt11 vectors was performed according to Sambrook et al.. The recombinant bacteriophages were amplified by growth in E. coli and the resulting library of 10⁷ cDNA clones were plaque immunoscreened as shown in Figure 1 for the presence of chronic NBNC hepatitis relating antigens by interaction with an antibody preparation derived from the pooled serum of the five chronic NBNC hepatitis patients. A positive clone, "K" was identified and purified by three successive rounds of plaque amplification and plaque immunoscreening. By the first round of screening 583 positive cDNA clones were picked up from screening plates. The second round of screening picked up 24 clearly positive clones and finally 4 clones were picked up. Clone "K" was selected from the 4 clones after test by a panel serum preparation consisting from patients with chronic hepatitis B, chronic hepatitis C and chronic NBNC hepatitis, and normal controls (5 each).

### Novel Nucleotide and Amino Acid Sequences of Clone-K

The nucleotide sequence of the cDNA insert of clone-K and the deduced amino acid sequence (hereinafter "K-polypeptide") encoded by the insert, are given in Figure 2. Neither the nucleotide sequence nor the amino acid sequence shows any homology to the nucleotide sequences of HBV or HCV reported in the scientific literature or in various patent applications. Neither do the clone-K DNA or amino acid sequences display any detectable homology with the available sequences of the hepatitis A, B, C, D, E, or G viruses, or any other sequences catalogued in the maj or data bases. Thus, clone-K represents a novel nucleic acid sequence encoding a novel polypeptide having one or more epitopes recognized by antibodies in patients displaying clinical and histological features of chronic NBNC hepatitis, consequently, acute and chronic NA-G hepatitis. As such, the agent may represent an additional contributing factor to the development of NA-G hepatitis in humans.

### None of the Classical Consensus Glycosylation Sites in Clone-K Nucleic Acid Sequence

The disclosed clone-K nucleic acid sequence displays none of the classical consensus glycosylation sites. This indicates that K-polypeptides expressed from eukaryotic and other host cells should possess the functional epitope characteristics of the disclosed K-polypeptide. Thus, unpredictability as to appropriate host cells as may be encountered with various proteins requiring specific glycosylation patterns should not be a factor with K-polypeptide sequences and related sequence.

### Preparation of Glutathione-S-Transferase (GST) Clone-K Fusion Protein

The insert of K-polypeptide was subcloned into an expression plasmid and expressed as a GST-fusionprotein in E. coli according to Sambrook et al., supra, pages A9 . 27-9 . 28 (2001). Plasmid pUC19 containing clone-K insert was digested with EcoR1 and the K fragment was subcloned into a variant of the plasmid vector pGEX-2T according to Gearing et al., EMBO J 8: 3667 (1989). This variant (designated pGEX-2Ta) was constructed by opening the pGEX-2T plasmid by digestion with BamH1 restriction endonuclease, filling the overhanging termini by the action of the Klenow fragment of E. coli DNA polymerase, and ligating the blunt ends together according to Sambrook et a1., supra, pages A6.4, 15.6-15.8 and A4.15-A4.17 (2001). The polypeptide encoded by clone-K was expressed from plasmid pGEX-2Ta as a glutathione-S-transferase (GST) fusionproteininE. coli strain DH5a according to methods of Smith et al. , Gene 67: 31 (1988). The GST-clone-K fusion protein was purified for western blot according to Sambrook et al., supra, pages 15.4-15.8 (2001).

### Identification of GST-clone-K Fusion Protein at a Predicted Molecular Size on SDS-PAGE

The purified GST-clone-K fusion protein supra was solubilized with detergents and reducing agents, and separated by performing size-fractionation in sodium dodecyl sulfate polyacrylamide gel electrophoresis (SDS-PAGE) using a Laemmli discontinuous buffer system according to Sambrook et al., supra, pages A8.40-A8.55 (2001) . The gel consisted of a 15% separating gel (pH 8.8) and 4.5% stacking gel (pH 6.8), was electrophoresed by applying purified GST-K-antigen in 2% SDS, 0.125M Tris pH 6.8, 10% glycerol, 1% 2-mercaptoethanol and 0.02% pyronin Y tracking dye boiled for 5 minutes in a water bath. The sample was applied as128µl aliquouts (2.7 mg each) to wells and electrophoresed at constant current until the tracking dye reached the bottom of the gel. Two wells (Lanes A and B) of the SDS-PAGE in Figure 2, Lane A is for low molecular weight marker and Lane B is for purified GST-clone-K fusion protein. They were stained by CBB to identify molecular size of GST-clone-K fusion protein. The apparent molecular weight of GST-clone-K run in well B was estimated by extrapolation from a standard plot of the logarithm of the molecular weight versus the electrophoretic mobility of rainbow molecular weight markers (Amersham Corp., Arlington Heights, IL) run in an adjacent well A, and was found to be identical to the predicted molecular weight (32KD) of the fusion protein.

### Western Blot to Identify GST-Clone-K Protein and Anti-K Antibodies

Western blot was conducted to identify GST-clone-K protein and specific antibodies against K-polypeptide according to Sambrook et al., supra, page A9.28 (2001).

One strongly reactive serum and 2 negative controls on plaque immunoscreening were run on SDS-PAGE to identify anti-K antibodies in the positive samples at a predicted molecular size of GST-clone-K fusion protein supra in Figure 2.

The purified GST-clone-K fusion protein was applied to three wells (Lanes C, D and E) on the gel plate for SDS-PAGE adjacent to well B in Figure 2. After electrophoresis, proteins in the gel (Lanes C, D and E) were transferred from the polyacrylamide gel electrophoresed to a solid support, polyvinylidene difluoride (PVDF) filter membranes.

The filter was subsequently air dried, cut into 3 pieces along the electrophoretic lanes and exposed separately to the 3 sera pre-treated with GST purified from bacterial cultures containing unmodified pGEX-2T lacking the K insert to absorb anti-GST antibodies in the serum. Lane C was for a serum positive for anti-K by plaque immunoscreening, presumably specific for the target K-polypeptide and the other 2 wells (Lanes D and E) were for 2 control sera negative for anti-K-polypeptide by plaque immunoscreening. Finally, the bound antibody is detected by the secondary immunological reagents, anti-IgG+IgM coupled to horseradish peroxidase, followed by enzymatic production of a colored precipitate. Consequently GST fusion K-polypeptide was localized by anti-K antibodies in a patient serum in the well Catan predicted molecular size (32KD) of the polypeptide (Figure 2). More precise information of the methods can be obtained from Sambrook et a1., supra, pages A 9.28 for review, A9.34-A9.37 for enzyme conjugated antibodies, A8.40-A8.45 for poly-acrylammide gel electrophoresis, A8.52-A8.53 for immunoblotting and A8.54-A8.55 (2001) for staining proteins during immunoblotting.

### Expression of K Nucleic Acid Sequence in Plasmid of E-coli for a Large Scale Preparattion of K-polypeptide

The dislosed K-nucleotide sequence has been cloned in both phage and plasmid vectors as descried in the Examples below. The K-polypeptide has been expressed from such vectors in E. coli (e. g. , other prokaryotic hosts, yeast and other eukaryotic hosts such as mammalian cells) also available to those skilled in the art. Sambrook et a1., supra, pages A3.1-A3.10 (2001).

The vectors usable for subcloning and/or expression of clone-K or related sequences include anyvectors into which a DNA sequence such as represented by clone-K can be inserted along with any other elements necessary for optimum transcription and translation of the insert sequence. Such vectors can be transferred into and replicated within one or more of the host cell types listed above. It is preferred that the vectors useful in the present invention possess some or all of the following characteristics:
(1) be stably maintainable in the host cells for extended periods ;
(2) be propagated at high copy number in the desired host cell;
(3) possess sequence capable of promoting transcription of the insert;
(4) possess sequence coding for a selectable trait such as drug resistance; and
(5) possess sequence capable of terminating transcription.

For expression, preferably the vectors include at least one promoter, at least one Shine-Delgarno sequence, Shine et al., Nature 254: 34 (1975) or like sequence and initiator codon, at least one terminator codon, and at least one sequence encoding a secretory leader or signal sequence. Any other elements necessary for transcription and/or translation of clone-K-related sequences may be added to the vectors using methods described in the available scientific literature. The general approaches and numerous examples for cloning and expression of insert sequences in vectors adapted for eukaryotic and prokaryotic hosts is provided in Sambrook et a1., supra, chapters 1-4, 7, 14-17 (2001). Yeast vectors may include the yeast artificial chromosome vectors as described, for example, in Burke et a1., Science 236: 806 (1987).

### Utility of Anti-K Test for Late Onset Chronic Transfusion Associated NA-G Hepatitis

The immunoassay results supra clearly indicate the immunodiagnostic utility of the clone-K-polypeptide as a specific marker for NA-G hepatitis disease. Thus, the K-polypeptide possesses at least one epitope diagnostic for NA-G hepatitis. On the basis of the results disclosed herein, the immunodiagnostic utility of the NA-G hepatitis-diagnostic epitope or epitopes of the K-polypeptide may be defined as detectable NA-G hepatitis immunoreactivity with serum from a percentage of individuals at greater number of positive for NA-G hepatitis patients than for random blood donors from a general population of donors. The blood pool used to construct the phage library from which clone-K was isolated was entirely human in origin. It is possible that clone-K is derived from an agent that has a host range restricted to humans. The incidence of acute transfusion associated hepatitis is approaching zero and is almost certainly under 0.5% per transfusion episode stated by Alter, Vox-Sang 67: Suppl 319 (1994). However a long term study showed that 30% of 129 living persons with previously diagnosed TAH were unrelated to hepatitis viruses A, B, C, and G, suggesting another unidentified agent in the patients in their chronic state, chronic NBNC hepatitis, probably, late onset NA-G hepatitis. Seeff L et al., Hepatology 33: 455 (2001). As shown infra, in Table 4 in EXANPLE 3, 12.5% of donors (n=40) with elevated ALT level but negative for HCV-RNA and HBV-DNA were positive for anti-K antibodies by plaque immunoscreening. Furthermore a study on maintenance hemodialysis patients shows, infra in Table 4 in EXAMPLE 3, that 10% of the patients having history of blood transfusion (n=100) were determined to be positive for anti-K by plaque immunoscreening while it is only 2.8% in the patients (n=72) without history of blood transfusion. In addition, in Italy and in Germany, as shown in Table 5 in EXAMPLE 3, 23.1% (n=13) and 10. 5% (n=38) of polytransfused patients were positive respectively for anti-K by plaque immunoscreening.

### Utility of Anti-K Test for Donated Blood to Prevent Transmission Associated NA-G Hepatitis

A close relation between blood transfusion and anti-K antibodies is clearly shown supra. Furthermore as shown infra in Table 3 in EXAPLE 3, one (1.7%) of 60 Japanese donors with normal ALT level and negative for both of HBV-DNA and HCV-RNA was positive for anti-K. In addition as shown infra, in Table 5 in EXAMPLE 3, 4 (2.7%) out of 156 Italian healthy controls were positive for ant-K. More surprisingly in Brazil, Czech Republic and Germany much higher rates, up to 12.5%, of anti-K antibodies by plaque immunoscreening were observed in blood donors as shown infra in the Table 5. These evidence show that anti-K test for blood donors must be useful to prevent transmission of anti-K related hepatitis, NA-G hepatitis.

### Utility of Anti-K Test to Diagnose Chronic NA-G Hepatitis

In 1998, 633 patients suffering from chronic viral hepatitis, liver cirrhosis and primary liver cancer visited the applicants' clinic as shown supra in Table 1. Liverhistologyof thesepatients was compatible with chronic viral hepatitis. In 85 (13.4%) of these patients, genomes of HBV and HCV were not detectable by PCR methods. Therefore these 85 patients should be included in chronic NBNC hepatitis, chronic NA-G hepatitis or chronic hepatitis of unknown etiology as among known hepatitis viruses only HBV and HCV cause chronic hepatitis. It should also be stressed that higher rates of anti-K antibodies are seen in patients with liver cirrhosis and primary liver cancer than in patients with chronic hepatitis supra in Table 1.

### Utility of Anti-K Test to Diagnose Acute NA-G Hepatitis

The known hepatitis viruses, A, B, C, D and E can cause acute hepatitis. Among them HDV needs HBV for its replication and HEV is seen in restricted areas. In dominant cases of acute hepatitis, HAV, HBV or HCV has usually been identified. However, as stated by Schiff, Schiff et al., eds., supra pages 719-724, approximately 17% of liver disease is still unexplained and it has been called as cryptogenic hepatitis, hepatitis of unknown etiology, NA-E hepatitis, NA-G hepatitis or NA-C hepatitis where HEV hepatitis is only rarely seen. As shown infra in Table 5 in EXAMPLE, anti-K antibodies were detected by plaque immunoscreening in 6.7% to 27.6% of patients with acute NA-G and acute NA-C hepatitis in acute phase. As the second antibody against human immunoglobulin contained anti-human IgG antibody and low titer of anti-human IgM antibody, by using a higher titer of anti-human-IgM-antibody the positive rate of anti-K in acute NA-G hepatitis can be raised.

### Enzyme Linked Immunoabsorbent Assay for Anti-K Test

In a further alternative embodiment, the K-polypeptide of the present invention may be coated onto microtiter plate wells in the classical enzyme linked immunoabsorbent assay (ELISA), incubated with sample, washed, and an enzyme-conjugated anti-human antiserum added. Glass fiber filters may also be utilized as the solid substrate in a radial partition chromatography format. Detection is conventionally carried out by adding the appropriate substrate/chromogen and measuring the resultant product. For a general discussion of ELISA see Belager et al., Klotz and Notermans, Langone et al. eds, Immunological Techniques, part D: Selected Immunoassay, Methods in Enzymology 84: 19-31, 194-201 and 223-238, respectively (1982).

### The Other Assays to Detect Anti-K

Further alternative assay formats applicable to the polypeptide of the present invention include without limitation to western blot as described above and as referenced in Towbin et a1., Proc Natl Acad Sci 76: 4350 (1979); radio-immunoassay, Sambrook et al., supra, pages A9.29-A9.30 (2001); competitive assays, Diamandis, Clin Biochem 21: 139 (1988); noncompetitive assays, Thoma et al., Eur J Biochem 123:613 (1982); immunoprecipitation, Tojo et a1. , Clin Chem 34: 2423 (1988) ; dot blots, Jahn et a1., Proc Natl Acad Sci USA 81: 1684 (1984) ; andparticleconcentration fluorescence immunoassay (PCFIA), Jolley et al., J Immuno1 Meth 67: 21 (1984).

### Chemical Synthesis of K-Nucleotide and K-polypeptide

In alternative embodiments, both the clone-K nucleic acid sequence and fragments thereof, as well as the K-polypeptide and fragments thereof, may be chemically synthesized using well-known procedures. For reviews on chemical synthesis of nucleic acids, see Sambrook et al., supra 10.42-10.46 (2001) and Itakura et a1., Ann Rev Biochem 53: 323-356 (1984). Polypeptides of desired sequence may be chemically synthesized on commercially available automated polypeptide synthesizers such as the Milligen-Biosearch Model 9600 Peptide Synthesizers.

K-polypeptide for Vaccine Development Against NA-G Hepatitis The K-polypeptide and fragments thereof also have potential utility as vaccines for the prevention, amelioration or treatment of chronic NBNC hepatitis and acute NA-G hepatitis. As documented in Examples 3 infra, the epitope or epitopes represented by the K-polypeptide have been shown to react with antibodies present in the serum of certain chronic NBNC hepatitis and acute NA-G hepatitis patients . It is therefore likely that the K-polypeptide or fragments thereof, coupled, if necessary, to appropriate carrier macromolecules will generate a NA-G hepatitis specific immune response in humans immunized with such polypeptides. Tam, Proc Natl Acad Sci USA 85 : 5409 (1988) and Hudecz et al., Bioconjug Chem. 10: 781 (1999). The K-polypeptide or appropriate fragments may be expressed from recombinant vectors or produced by chemical synthesis, as isolated polypeptide or as a component of a fusion protein. The polypeptide or fusion protein may be administered to humans in a pharmaceutically acceptable carrier known to those skilled in the art in order to elicit an immune response directed against the etiologic agent represented by the K-polypeptide.

### K-polypeptide for Development of Passive Immunization Therapies for NA-G Hepatitis

The K-polypeptide also may be used to generate, using known procedures, materials that could function as components of passive immunization therapies. For example, the K-polypeptide or fragments thereof may be employed to generate polyclonal or monoclonal, antibodies directed against the agent represented by the K-polypeptide. Such antibodies, or immunoreactive portions thereof, can be used to impart immunity to the K-agent by direct injection in a pharmaceutically acceptable carrier into a patient's blood stream. The rationale for passive immunization is that the injected antibodies, independently of the patient's endogenous immune system, bind to and facilitate inactivation and removal of the disease causing agent. Passive immunization has been found effective in conferring at least transient immunity to a wide array of disease agents. For example, passive immunization with monoclonal antibodies has conferred resistance to several of the viruses. Schlesinger et a1., Laskey ed., Technological Advances in Vaccine Development, pages 11-20 (1988). Similarly, passive immunization with polyclonal antibodies has conferred resistance to simian immunodeficiency virus and human immunodeficiency virus type 2. Putkonen et al., Nature 352: 436 (1991).

### Monoclonal Antibodies Targeting K-Polypeptide

Methods for producing polyclonal and monoclonal antibodies with polypeptide starting materials are well established in the scientific literature. For example, the immunoglobulin fraction of a subject immunized with K-polypeptide could be affinity purified on a column containing K-polypeptide bound to a solid phase. This would provide a purified preparation of polyclonal antibodies directed against the K-polypeptide. Likewise the K-polypeptide could be used to generate hybridoma cell lines secreting monolonal antibodies directed against the K-polypeptide. Suchhybridomascanbegenerated, and the secreted monoclonal antibodies isolated and purified, using well-known methods as described, for example, in Kohler, Science 233: 1281 (1986).

### Probes to Detect Clone-K Related Nucleic Acids in Patient Serum or Tissues

The disclosed nucleic acid sequence of clone-K also will be useful in a variety of assay formats utilizing nucleic acid targets. The clone-K nucleic acid sequence, for example, may be used as a probe to detect the presence of clone-K-related nucleic acids in patient serum or tissues. The clone-K sequence, or a fragment thereof, is labeled with an appropriate radioactive tag (e.g., ³²p) or with an appropriate non-radioactive tag (e.g., biotin) and hybridized to either amplified or unamplified nucleic acid from patient serum or body tissue. For amplification of nucleic acid in patient serum or body tissue, any of the amplification systems known to those skilled in the art may be employed. For example, primers based on the disclosed clone-K sequence may be synthesized and used in the polymerase chain reaction (PCR) to amplify DNA or cDNA derived from RNA extracted from serum, cell or tissue. The various steps in performing PCR are described in Sambrook et al., supra, pages 8.1-8.126 (2001).

### In Situ Hybridization of K-Nucleotide for Detection of Clone-K Related NA-G Hepatitis Agents

Hybridization to amplified or unamplified nucleic acid can be accomplished in situ or with extracted nucleic acids, and detected with liquid scintillation counting, autoradiography, or appropriate techniques for detecting non-radioactive tags. For a review of nucleic acid hybridization techniques, see Sambrook et a1., supra, pages 9.35, 9.73-9.81 and 10.1-10.10 (2001). It is to be understood that an RNA sequence corresponding to the sequence disclosed in Figure 2A may be manufactured with procedures available to those skilled in the art and used as a RNA probe in appropriate circumstances.

### Standard Site-Specific Mutagenesis Procedures or Other Techniques for Clone-K Nucleotide Sequence

The entire clone-K sequence, or any appropriately diagnostic fragment thereof capable of forming a detectable hybrid with a target nucleic acid, may be used for nucleic acid hybridization as described above. Likewise, it is to be understood that the clone-K sequence readily may be altered with standard site-specific mutagenesis procedures or other techniques, see Sambrook et a1., supra, pages 13.2-13.10 (2001), so as to provide an array of probes useful in detecting clone-K-related NA-G hepatitis agents that have undergone mutational variation. It is known, for example, that many RNA viruses are hypervariable, and it may be necessary to use known methods as referenced above to provide clone-K-related sequences adapted for optimum detection of such variants. Generally any nucleic acid sequence capable of hybridizing to the disclosed clone-K sequence under low-to-moderate stringency washing conditions that is, under combinations of temperature and salt concentration approximately 20°C-30°C below the calculated melting temperature Tm of a perfectly matched K-K hybrid, see Sambrook et al., supra, pages 10.2-10.6 and 10.47-10.48 (2001), will have potential utility as a diagnostic probe for the agent represented by clone-K.

### Nucleotide Sequences Altered at Various Codon Third Position of K-Nucleotide and Minor Change in K-polypeptide

In like manner, the disclosed clone-K nucleic acid sequence may be altered at various codon third positions in accordance with the degeneracy of the genetic code without altering the encoded amino acid sequence. Sambrook et al., supra, page 15.12 (2001). On the other hand, it is to be emphasized that minor changes in amino acid sequence (e.g., substitutions, additions or deletions) may not appreciably affect assay performance because the epitope or epitopes represented by the disclosed K-polypeptide are not changed to a degree that destroys the immunodiagnostic utility of such an altered polypeptide in an assay for the K agent. As such, polypeptides and corresponding eptiopes having such minor changes in structure are considered to be substantially similar to, or equivalents of, polypeptides and epitopes having strict homology to the polypeptide and epitope(s) encoded by the disclosed clone-K nucleic acid sequence.

Extension of K-Nucleotide Sequence to Enhance Diagnostic Utility Additional nucleic acid sequences representing genomic sequences flanking the genomic sequence corresponding to the disclosed clone-K-nucleotide sequence are readily obtained using standard procedures. Such sequences, whether 5' or 3' to the K sequence, may enhance the diagnostic utility of the disclosed clone-K nucleic acid sequence or may have separate utility as nucleic acid probes. In addition, such sequences may encode amino acid sequence enhancing the diagnostic utility of the disclosed K-polypeptide, or may encode additional NA-G hepatitis epitopes having independent immunodiagnostic utility. Methods for identifying and isolating such flanking nucleic acid sequences (chromosome "crawling" or "walking") are well known to those skilled in the art. For reviews of various procedures for isolating flanking sequences, see Sambrook et a1., supra, pages 4.8-4.10 and 13.15-13.18 (2001). For example, the λgt11 library referenced above and described in the Examples provided infra may be re-screened with oligonucleotide probe specific for the 5' or 3' end portions of the disclosed clone-K sequence. As such, clones overlapping with the originally isolated clone-K may be isolated and will contain nucleic acid sequences flanking the disclosed clone-K sequence. Alternatively, oligonucleotides based on the disclosed clone-K sequence may be used as primers to generate new cDNA libraries from which additional flanking clones may be isolated.

The present invention will now be described in detail with reference to the following Examples, which should not be construed to be limiting the scope of the present invention.

### Brief description of the figures.

Figure 1 represents a result of the plaque immunoscreening.
Figure 2 represents a result of the identification of anti-K antibody by western blotting.

### EXAMPLE 1

### Isolation and DNA Characterization of Clone-K

During plaque immunoscreening of a cDNA library prepared from pooled peripheral blood mononuclear cells (PBMC) by NYCOMED PHARMAAS Diagnostics (Oslo, Norway), plasma or serum of chronic NBNC hepatitis patients, a phage clone (K) reactive with sera from chronic NBNC hepatitis patients was identified by use of the following method:
Step 1: One ml of Lymphoprep (NYCOMED PHARMA AS Diagnostics, Oslo, Norway) was added to the mononuclear cell fraction prepared from 10mL heparinized peripheral blood according to Ting et al., Vox Sang 20: 561 (1971) and RNA was extracted by the manufacturer's instruction. Plasma and serum RNA was extracted by using TRIZOL-LS (GIBCO BRL, Life Technology, Gaithersburg, MD, USA) according to the manufacturer's instruction.
Step 2: Conversion to cDNA and construction of double stranded cDNA library was accomplished by GIBCO-BRL Choice System. After attachment of EcoRI linkers, the cDNAs were ligated into λgt11 arms (Stratagene) and packaged into phage heads (Gigapack III Gold; Stratagene: La Jolla, CA, USA). The resulting phage library represented approximately 10 million clones, of which approximately 66% contained inserts.
Step 3: A 100 ml culture of E. coli strain Y1090 was grown to an optical density of 0.5 at 600nm wavelength in medium composed of LB broth containing 50µg/ml ampicillin, 0.2% w/v maltose and 10mM MgSO4. Bacteria were then pelleted by centrifugation, and resuspended in 9.2ml of ice-cold 10mM MgSO₄.
   Bacteriophage suspensions representing 50,000 plaque-forming units in 300ml each of SM buffer (50mM Tris-HCl pH 7.4, 100mM NaCl, 10mM MgSO₄, w/v gelatin) were mixed with 450ml each of the bacterial suspension prepared above, and were agitated at 150 rpm for 10 minutes at 37°C. Tubes were then warmed to 47°C, and 9ml of top agarose (0.75% w/v in LBbroth containing ampicillin at 50µg/ml) was added to each tube of bacteria with phage. These tubes were then poured onto the surface of 150mm diameter plates of LB agar containing ampicillin (50µg/ml), allowed to harden, and incubated in an invertedposi tion at 42°C . After approximately three hours of incubation, phage plaques became visible. The plates were then covered with 137mm diameter nitrocellulose filters that had been previously soaked in a solution of low isopropylthio-beta-galactoside (Sigma, St. Louis, MO, USA) and allowed to dry. The plates were incubated for a further three hours at 37°C, and the membrane orientation marked by piercing the membrane and bacteriological medium several times with an ink-covered needle. Membranes were then removed from the plates, washed with distilled water, and set aside to dry. Dry filters were then incubated in a solution of 2% w/v non-fat dry milk in lx TBS (0.1M Tris pH 7.4, 0.5M NaC1, 0.1% w/vNaN₃) for 15 to 60 minutes at room temperature.
Step 4: Plasmas or sera (10ml each) from five individuals suspected to have suffered from chronic NANB hepatitis were pooled, and pre-adsorbed to an equal volume of E. coli. Y1090-lysate. The lysate was prepared by three rounds of homogenization of the bacterial cell pellet derived from a 4 liter culture. The mixture was centrifuged at 7100 x g for 10 minutes, and 4ml of supernatant (equivalent to 10ml of untreated pooled plasma) was diluted with 960ml of 5% w/v non-fat dry milk in 1 x TBS.
Step 5 : Membranes from Step 3 were aspirated to remove themilk/TBS solution, and 20ml of treated antibody from Step 4 was added to each filter. Membranes were incubated with the antibody solution overnight at room temperature with gentle rocking. Antibody solution was then removed by aspiration, and membranes were washed for 30 minutes with 6 changes of TBS/Tween solution.

Horseraddish peroxidase-conjugated goat anti-human IgG+IgM (Cappel/ICN) was diluted 1: 3500 in lx TBS containing 2% non-fat dry milk. Membranes were incubated in 20ml each of this diluted secondary antibody suspension for 2.5 hours at room temperature, and then washed with six changes of 1xTBS/Tween 20. Following the final wash, substrate solution (10mM Tris-HCl, pH6.5, 150mM NaCl) containing 500µg/ml of 4-chloro-1-naphtol (Sigma, St. Louis, MO, USA) was added to the filters, and incubated until reactive plaques had darkened (2-7 minutes) . Reactions were then terminated by a water rinse.

Reactive plaques were identified as darkly staining blue circles of approximately 1 mm diameter, often exhibiting a "doughnut" like appearance. One clone, identified as K, was found to be reactive to pooled serum from chronic NBNC hepatitis patients. This bacteriophage plaque was picked up by aligning the membrane with the bacteriological plate, and an agarose plug from the plate was removed using the wide end of a Pasteur pipette. Phage in the plug were eluted overnight by incubation at 4°C in 1ml of SM buffer containing 50µl chloroform. The bacteriophage clone was enriched and purified by three successive rounds of plaque immunoscreeing as described supra, this example.

As described in Figure 1 (C), membrane containing an equal mixture of E. coli infected with λgt11 clone-K and wild-type λgt11 was cut into 2X3 cm peaces to detect anti-K-polypeptide (plaque immunoscreening). The distribution of anti-K-polypeptide in patients with chronic hepatitis B, C and NBNC, blood donors, patients with maintenance hemodialysis and multi-transfused patients are shown infra in Tables 2, 3, 4 and 5.

### EXAMPLE 2

### DNA and Encoded Amino Acid Sequence of Clone-K

To determine the DNA sequence of the recombinant insert contained within phage clone-K, the following steps were performed.

Step 1: The recombinant DNA insert from phage clone-K selected by immunoscreening was amplified by PCR using primers forward primer : GGTGG CGACG ACTCC TGGAG CCCG, and reverse primer: TTGAC ACCAG ACCAA CTGGT AATG, that flank the EcoRI site of λgt11. The amplified double-strands DNA was labeled by the forward primer with cycle sequencing using ABI PRISM BigDye Terminator Cycle Sequencing Ready Reaction Kit (PE Applied Biosystems, Foster City, CA, USA) . The labeled DNA was electrophoresed and analyzed by ABI PRISM 310 and Sequence Navigator Software (PE Applied Biosystems, Foster City, CA, USA).

The recombinant DNA sequence of clone-K determined by the above methods, and the deduced amino acid sequence encoded by it, are shown in Figure 2, infra. The sequences display none of the classical N-linked or O-linked glycosylation sites.

Analysis of this DNA and its encoded protein sequence shows no detectable levels of homology with sequences related to HAV, HBV, HCV, HDV, HEV and HGV reported in the available scientific literature or in various patent applications. Neither do the clone-K DNA or amino acid sequences display detectable homology with the available sequences of the hepatitis A, B, C, D, E and G viruses, with the sequences published in the articles and patent application of Arima et a1., supra, or with any other sequences entered in the GenBank and EMBL data bases as searched on December 27, 2001.

### EXAMPLE 3

### Plaque Immunoscreening for Detection of Anti-K Antibodies in Human Serum

The clone-K antigen was tested by plaque immunoscreening for immunoreactivity to serum from additional patients with acute NA-G hepatitis, chronic NBNC (equivalent to NA-G) hepatitis, maintenance hemodialysis and history of multi-blood-transfusion. Serum from chronic hepatitis B patients, chronic hepatitis C patients, and healthy persons were included in the test as controls.

For plaque immunoscreening, clone-K-polypeptide expressed in the plaques was transferred to and immobilized on a filter membrane. The bound protein was then reacted with test serum under conditions allowing binding of any anti-K antibodies to the immobilized protein. Binding of anti-K antibodies was detected with enzyme-labeled signal antibodies. The signal antibodies comprised horseradish peroxidase-conjugated goat anti-human IgG + IgM. Reaction with an appropriate chromogenic substrate for horseradish peroxidase then provided a visual indicator of the presence or absence of anti-K antibodies in a particular patient test sample as shown in Figure 1 (C). In this immunodiagnostic format, a positive test is indicative of the presence of a patient's anti-K-polypeptide antibody sandwiched between the immobilized K-polypeptide and the signal antibody. In the absence of anti-K-polypeptide antibody, the antigen-antibody-signal antibody complex does not form and no chromogenic reaction above background is detected.

As demonstrated infra Tables 2,3,4 and 5, the epitope or epitopes represented in the K-polypeptide have marked diagnostic utility for chronic NBNC hepatitis, consequently for acute NA-G hepatitis, and particularly for screening test of donated blood. In applicants' clinic, 72.0% of 25 patients with chronic NBNC hepatitis possessed detectable antibodies reactive with the K-polypeptide when tested by plaque immunoscleening while, in contrast, none of 30 patients with chronic hepatitis B and only 2 of 110 (1.8%) of patients with chronic hepatitis C possessed such detectable anti-K antibodies (Table 2). In addition, it should be stressed that the latter 2 patients with chronic hepatitis C positive for anti-K have history of blood transfusion.

**Table 2**

| Anti-K in Patients with Chronic Hepatitis B, C and NBNC | | |
|---|---|---|
| | Number of Patients | |
| Chronic Hepatitis* | Patients | Positive for anti-K |
| NBNC | 25 | 18 (72.0%) |
| B | 30 | 0 ( 0%) |
| C | 110 | 2** ( 1.8%) |

| | | |
|---|---|---|
| *Histology proven chronic hepatitis. | | |
| **Having history of blood transfusion. | | |

Then to see whether anti-K is present in Japanese general population, random blood donors negative for HBV-DNA, HCV-RNA, HIV-RNA and HTLV-1-RNA were tested for anti-K by plaque immunoscleening (Table 3). Only one (1.7%) out of 60 donors with ALT in the reference range is positive for anti-K. On the contrary, an appreciable numbers, five (12.5%) out of 40 blood donors with ALT over the reference range, are positive for anti-K suggesting the presence of infection relating to an exogenous K-antigen in the asymptomatic general population.

**Table 3**

| Anti-K in Japanese Blood Donors* | | |
|---|---|---|
| | Number of Patients | |
| ALT Level | Patients | Positive for Anti-K |
| Within Reference Range | 60 | 1 (1.7%) |
| Elevated | 40 | 5 (12.5%) |

| | | |
|---|---|---|
| *PCR negative for HBV-DNA, HCV-RNA, HIV-RNA and HTLV-1-RNA. | | |

As demonstrated supra, hepatitis relating to anti-K seems to be blood transmitted, 172 patients with maintenance hemodialysis, thought to be at high risk for blood transmitted diseases, were tested for anti-K by plaque immunoscreening (Table 4). Patients were divided into two groups by their start of hemodialysis before (n=69) or after (n=103) 1990 when the first generation of anti-HCV test was initiated by Japanese Blood Center, then they were subdivided by history of blood transfusion to study influence of the anti-HCV test and blood transfusion to the present status of anti-K and HCV-RNA in their serum. They were tested for HBV-DNA andHCV-RNA. All of them were negative forHIV-RNAandHTLV-1-RNA.
Two patients, one started hemodialysis in 1994 without history of blood transfusion and the other one started hemodialysis in 1975 having history of blood transfusion, are positive for HBV-DNA but negative for both anti-K and HCV-RNA. Two out of 6 patients with abnormally elevated serum ALT level are positive for HCV-RNA but none of them is positive for anti-K. Overall only one out of 13 HCV-RNA carriers were positive for anti-K suggesting rare co-infection of hepatitis C and anti-K-relating hepatitis. Ten (10%) and 12 (12%) out of 100 patients having history of blood transfusion are positive for anti-K and HCV-RNA respectively while only 2 (2.8%) and 1 (1.4%) out of 72 patients without history of blood transfusion are positive for anti-K and HCV-RNA respectively. The evidence suggests blood transmission of anti-K relating hepatitis as well as hepatitis C which is well known as a blood transmitted disease. In the patients having history of blood transfusion, by the introduction of anti-HCV test for donated blood caused a reduction of HCV-RNA positive patients from 10/53 (18.9%) to 2/47(4.3%). On the contrary the positive rate of anti-K increased slightly from 9.4% to 10.6% in the same period. Furthermore in the whole patients, the introduction of the anti-HCV test reduced HCV positive patients from 15.9% to 1.9% while the reduction in anti-Kpositive patients was from 8.7% to 5.8% suggesting only rare correlation between transmissions of HCV and K-relating hepatitis agent.

**Table 4**

| Influence of Anti-HCV Test* for Donated Blood and Blood Transfusion to Anti-K and HCV-RNA in Japanese Patients with Maintenance Hemodialysis. | | | |
|---|---|---|---|
| Start of Hemodialysis | | | |
| History of Blood Transfusion | Before 1990 | After 1990 | Total |
| Yes | n=53 | n=47 | n=100 |
| Anti-K (+) | 5( 9.4%) | 5(10.6%) | 10(10.0%) |
| HCV-RNA (+) | 10(18.9%) | 2( 4.3%) | 12(12.0%) |
| Anti-K (+)/HCV-RNA (+) | 1( 1.9%) | 0 | 0 |
| No | n=16 | n=56 | n=72 |
| Anti-K (+) | 1( 6.3%) 1( | 1.8%) | 2( 2.8%) |
| HCV-RNA (+) | 1( 6.3%) | | 1( 1.4%) |
| Anti-K (+)/HCV-RNA (+) | 0 | 0 | 0 |
| Total | n=69 | n=103 | n=172 |
| Anti-K (+) | 6( 8.7%) | 6( 5.8%) | 12 ( 7.0%) |
| HCV-RNA (+) | 11(15.9%) | 2( 1.9%) | 13( 7.6%) |
| Anti-K (+)/HCV-RNA (+) | 1( 1.4%) | 0 | 0 |

| | | | |
|---|---|---|---|
| *Anti-HCV test was initiated by Japanese Blood Bank in the beginning of 1990 | | | |

By plaque immunoscleening, worldwide distribution of anti-K was confirmed in serum samples provided by researches in Japan (see Tables 2, 3 and 4), Taiwan, Korea, Brazil, Germany, Italy, Greece and Czech as shown infra (Table 5). It should be stressed that eight out of 29 (27.6%) patients with acute NA-G hepatitis in the acute phase in Taiwan were positive for anti-K of which increase can be expected to in their convalescent stage.

Anti-K is detected in patients with chronic NBNC disease, 2 (16.7%) out of 12 patients from Italy and 7 (33.3%) out of 21 patients from Germany. In polytransfused patients, 3 (23.1%) out of 13 patients from Italy and 4 (10.5%) out of 38 patients from Germany are positive. In addition, 8 (12.5%) out of 64 German blood donors and 4 (2.7%) out of 156 Italian healthy controls are positive for such antibodies against K-polypeptide. These evidence suggest worldwide distribution of anti-K positive persons particularly patients with liver diseases and polytransfusion, and also blood donors and healthy controls. Known hepatitis viruses are detected in blood donors and healthy controls as described supra, by Sjogren et al., Chan et al., Rizzeto et al. , Krawczynki et al. and Schiff et al. , pages 745-867 (1998).

**Table 5**

| Anti-K Blindly Tested in Patients with Acute NA-G Hepatitis, Chronic NBNC Liver Diseases and Polytransfusion, and Blood Donors. | | |
|---|---|---|
| | Number of | |
| | Subjects | Positive (%) |
| Acute NonA-G Hepatitis | 29 | 8 (27.6)¹ |
| Acute NonA-C Hepatitis | 30 | 5 (16.7)² |
| | 30 | 2 ( 6.7)³ |
| Non-B Non-C Chronic Liver Disease | 12 | 2 (16.7)⁴ |
| | 21 | 7 (33.3)⁵ |
| Polytransfused Patients | 13 | 3 (23.1)⁴ |
| | 38 | 4 (10.5)⁵ |
| Blood Donors | 60 | 6 (10.0)² |
| | 30 | 2 ( 6.7)³ |
| | 64 | 8 (12.5)⁵ |
| Healthy Controls | 156 | 4 ( 2.7)⁴ |
| Origin not Informed | 209 | 27 (12.9)⁶ |
| | 119 | 2 ( 1.7)⁷ |

Samples were kindly provided by ¹Dr . Yun-Fun Liaw, Liver Research Unit, Chang Gung Memorial Hospital, Chang Gung University, Taipei, Taiwan, ²Dr. Christian Niel, Dept of Virology, Fundacao Oswaldo Cruz, Rio de Janeiro, Brazil, ³Dr. Vladimir Strakrle, Dept of Infectious Deseases, Custody Hospital, Brno, Czech Republic, ⁴Dr. Gabriele Pozzato, Ospadale Cattinara, Medicina Interna, Trieste, Italy, ⁵Dr. Sergei Viazov, Institut fur Virologie, Uniklinikum Essen, Essen, Germany, ⁶Dr. Jongyoung Choi, Dept of InternMed, Catholic UniversityMedical College, Seoul, Korea and ⁷Dr. Anj elos Hatzakis, National Retrovirus Reference Center, Athens University Medical School, Athens, Greece.

To map antigenic epitope or epitopes, partly overlapping synthetic peptides corresponding to 5 regions of K peptide (69 residues): residues 1-22 (K1), 11-34 (K2), 23-46 (K3), 35-58 (K4) and 47-69 (K5) were tested by sera from 40 patients with chronic NBNC hepatitis. An enzyme immunoassay employed for the test revealed that only both K1 and K2 reacted to the same 6 (30%) out of 20 patients positive for anti-K by plaque immunoscreening. None of the rest 20 patients negative for anti-K by plaque immunoscreening reacted to them. Furthermore none of the 40 patients reacted to K3, K4, and K5. These results suggest that at least one epitope is present in the sequence of amino acid residues 11-22 of K peptide.

Studies to find out more fine epitope map using overlapping synthetic 12-mer peptides with three and then one amino-acid offsets revealed that a sequence amino acid 9 to 20 seems to comprise at least an important epitope of clone K peptide.

The foregoing detailed description has been provided for a better understanding of the invention only and no unnecessary limitation should be understood therefore as some modifications will be apparent to those skilled in the art without deviating from the spirit and scope of the appended claims.

### REFERENCES

1: Schiff, Diseses of the Liver. Schiff et al. eds., Philadelphia and New York; Lippincott-Raven, pages 719-724 (1998).
2: Doo et al. , Schiff et al. eds., supra, pages 725-744 (1998).
3: Tan et al., J Infect Dis 179: 1055 (1999).
4: Sjogren, Chan et al., Davis, Rizzetto et al., Krawczynski etal. and Schiff, Schiffetal. eds., supra, pages745-869 (1998).
5: Skidmore et al., J Med Virol 10: 223 (1982).
6: Hollinger et al., JAMA 250: 2313 (1983).
7: Mannucci et al., Ann Intern Med 120: 1 (1994).
8: Lemos et al., J Clin Virol 16: 71 (2000).
9: Chauhan et al., Lancet 341: 149 (1993).
10: Irshad et al., Trop Gastroenterol 18: 45 (1997).
11 in 4: Schiff et al. , Schiff et al. eds., supra, pages 861-867 (1998).
12: Purcell, Proc Natl Acad Sci USA 91: 2401 (1994).
13: Binotto et al., Aust N Z J Med 30: 668 (2000).
14: Alter, Vox-Sang 67 Suppl: 319 (1994).
15: Gerlich et al., J Viral Hepat 6 Suppl: 16 (1999).
16: Seeff et al., Hepatology 33: 455 (2001).
17: Matsumoto et al., Hepatology 30: 283 (1999).
18: Forns et al., J Med Virol 59: 313 (1999).
19: Umemura et al., Hepatology 33: 1303 (2001).
20: Shibata et al., J Infect Dis 184: 400 (2001).
21: Bosch, Okuda et al. eds., Liver Cancer. New York; Churchill-Livingstone, pages 13-28 (1997).
22 in 4: Chan et al. , Schiff et al. , eds., supra, pages 757-791 (1998).
23 in 4: Davis, Schiff et al. , eds. , supra, pages 793-836 (1998).
24: Leonhardt et al., Eur J Surg 165: 539 (1999).
25: Muller et al., Hepatology 25: 896 (1997).
26: Ishikawa et al., Jpn J Clin Oncol 28: 723 (1998).
27: Alter, Am J Med 107: 16S (1999).
28: Choo et a1., Science 244: 359 (1989)
29: Arima and Fukai, EPO Application No. 89309261.9 (1989).
30: Arima et al., Gastroenterology Jpn 24: 540 (1989).
31: Arima et al., Gastroenterology Jpn 24: 545 (1989).
32: Arima et al., Gastroenterology Jpn 24: 685 (1989).
33: Arima et al., Gastroenterology Jpn 25: 218 (1990).
34: Ting et al., Vox Sang 20: 561 (1971).
35: Sambrook et al., Molecular Cloning: A Laboratory Manual (3rd Ed.), Cold Spring Harbor Laboratory Press, Cold Spring Harbor, NY., pages 7.9-7.12 (2001).
36: Sambrook et al., supra, pages 11.6-11.9 (2001).
37: Sambrook et al., supra, pages 11.113-11.114 (2001).
38: Sambrook et al., supra, pages 14.8-14.11 (2001).
39: Sambrook et al., supra, pages A9.27-9.28 (2001).
40: Gearing et al., EMBO J 8: 3667 (1989).
41: Sambrook et al., supra, pages A6.4 (2001).
42: Sambrook et al., supra, pages 15.6-15.8 (2001).
43: Sambrook et al., supra, pages A4.15-A4.17 (2001).
44: Smith et al., Gene 67: 31 (1988).
45 Sambrook et al., supra, pages 15.4-15.8 (2001).
46: Sambrook et al., supra, pages A8.40-A8.55 (2001).
47: Sambrook et al., supra, page A9.28 (2001).
48: Sambrook et al., supra, pages A 9.28 (2001)
49: Sambrook et al., supra, pages A9.34-A9.37 (2001).
50: Sambrook et al., supra, pages A8.40-A8.45 (2001).
51: Sambrook et al., supra, pages A8.52-A8.53 (2001).
52: Sambrook et al., supra, pages A8.54-A8.55 (2001).
53: Sambrook et al., supra, pages A3.1-A3.10 (2001).
54: Shine et al., Nature 254: 34 (1975).
55: Sambrook et al., supra, chapters 1-4, 7, 14-17 (2001).
56: Burke et al., Science 236: 806 (1987).
57: Alter, Vox-Sang 67: Suppl 319 (1994).
58: Seeff L et al., Hepatology 33: 455 (2001).
59 in 4: Sjogren, Chan et al., Davis, Rizzetto et al. , Krawczynski etal. and Schiff, Schiff etal. eds., supra, pages 745-869 (1998).
60 and 61: Belanger et al., Klotz and Notermans, Langone et al. eds, Immunological Techniques, part D: Selected Immunoassay, Methods in Enzymology 84: 19-31, 194-201 and 223-238, respectively (1982).
62: Towbin et al., Proc Nat1 Acad Sci 76: 4350 (1979).
63: Sambrook et al., supra, pages A9.29-A9.30 (2001).
64: Diamandis, Clin Biochem 21: 139 (1988).
65: Thoma et al., Eur J Biochem 123:613 (1982).
66: Tojo et al., Clin Chem 34: 2423 (1988).
67: Jahn et al., Proc Nat1 Acad Sci USA 81: 1684 (1984).
68: Jolley et al., J Immunol Meth 67: 21 (1984).
69: Sambrook et al., supra, 10.42-10.46 (2001).
70: Itakura et al. Ann Rev Biochem 53:323 (1984).
71: Tam, Proc Natl Acad Sci USA 85: 5409 (1988).
72: Hudecz et al., Bioconjug Chem. 10: 781 (1999).
73: Schledinger et al., Laskey ed., Technological Advances in Vaccine Development, pages 11-20 (1988).
74: Putkonen et al., Nature 352: 436 (1991).
75: Kohler, Science 233: 1281 (1986).
76: Sambrook et al, supra pages 8. 1-8.126 (2001)
77: Sambrook et al. , supra, pages 9.35, 9.73-9.81 and 10.1-10.10 (2001).
78: Sambrook et al., supra, pages 13.2-13.10 (2001).
79: Sambrook et al., supra, pages 10.2-10.6 and 10.47-10.48 (2001).
80: Sambrook et al., supra, page 15.12 (2001).
81: Sambrook et al., supra, pages 4.8-4.10 and 13.15-13.18 (2001).
82: Ting et al., Vox Sang 20: 561 (1971).
83 in 4 : Sjogren, Chan et al . , Davis, Rizzetto et al., Krawczynski etal. and Schiff, Schiff etal. eds. , supra, pages 745-869 (1998).

## Claims

1. A purified and isolated DNA molecule comprising the nucleotide sequence as defined by SEQ ID No. 2.

2. A purified and isolated DNA molecule comprising a DNA sequence encoding a polypeptide having the immunodiagnostic utility of the polypeptide as defined in the Sequence Listing by SEQ ID NO: 1.

3. The purified and isolated DNA molecule of claim 1 wherein said DNA sequence comprises a DNA sequence encoding the polypeptide as defined in the Sequence Listing by SEQ ID NO: 1.

4. A purified and isolated DNA molecule comprising a DNA sequence encoding a polypeptide having an epitope substantially similar to a NA-G hepatitis-diagnostic epitope in the amino acid sequence defined in the Sequence Listing by SEQ ID NO: 1.

5. A polypeptide comprising the amino acid sequence as defined by SEQ ID No. 1.

6. A polypeptide comprising an amino acid sequence having the immunodiagnostic utility of the polypeptide as defined in the Sequence Listing by SEQ ID NO: 1.

7. A polypeptide comprising an amino acid sequence having an epitope substantially similar to a NA-G hepatitis diagnostic epitope in the polypeptide as defined in the Sequence Listing by SEQ ID NO: 1.

8. A polypeptide comprising the amino acid sequence of the polypeptide as defined in the Sequence Listing by SEQ ID NO: 1.

9. A vector comprising the DNA sequence of claim 1.

10. A vector comprising the DNA sequence of claim 3.

11. A host cell transformed with the vector of claim 7 or 8.

12. The host cell of claim 9 wherein said host cell is a prokaryotic cell.

13. The host cell of claim 9 wherein said host cell is a eukaryotic cell.

14. A process for preparation of a polypeptide having the immunodiagnostic utility of the polypeptide as defined in the Sequence Listing by SEQ ID NO: 1 comprising: culturing the transformed host cells of claim 9 under conditions suitable for expression of said polypeptide, and recovering said polypeptide.

15. A process for preparation of a polypeptide having the immunodiagnostic utility of the polypeptide as defined in the Sequence Listing by SEQ IDNO: 1 comprising: chemical synthesis of said polypeptide.

16. A method of diagnosing NANH comprising: contacting serum from a patient with a polypeptide having the immunodiagnostic utility of the polypeptide as defined in the Sequence Listing by SEQ ID NO: 1; and determining whether or not said serum includes components immunoreacive with said polypeptide.

17. A method for screening blood for transfusion comprising isolating serum from said blood; contacting said serum with a polypeptide having the immnodiagnostic utility of the polypeptide as defined in the Sequence Listing by SEQ ID NO: 1; determining whether or not said serum includes components immunoreactive with said polypeptide; and effecting classification and handling of said blood based on said determination.

18. A vaccine for the prevention, amelioration or treatment of NA-G hepatitis, comprising a polypeptide having the immunodiagnostic utility of the polypeptide as defined in the Sequence Listing by SEQ ID NO:1, and a pharmaceutically acceptable carrier.

19. A purified and isolated polypeptide, said polypeptide comprising an antigen binding site of an antibody, said antigen binding site having specificity for an eptitope substantially similar to a NA-GH-diagnostic epitope in the polypeptide as defined in the Sequence Listing by SEQ ID NO: 1.

20. The polypeptide of claim 16, wherein said polypeptide is a monoclonal antibody.

21. A composition for passive immunization against NA-G hepatitis, comprising the polypeptide of claim 16 and a pharmaceutically acceptable carrier.
